# EUROPEAN PATENT APPLICATION

(11) **EP 2 799 232 A1**
(43) Date of publication of application: **05.11.2014**
(21) Application number: 13165985.6
(22) Date of filing: 30.04.2013
(51) Int. Cl.: B32B 27/32

(54) **Flexible package**

(71) Applicant: The Procter & Gamble Company, Cincinnati, OH 45202 (US)
(72) Inventor: Hawighorst, Joachim, 48599 Gronau (DE); Caruso, Pier-Lorenzo, 65824 Schwalbach am Taunus (DE)
(74) Representative: Boubel, Thomas

(57) **Abstract**

Flexible package comprising a co-extruded multi-layer film with high amount of renewable material exhibiting good sliding and sealability properties.

## Description

### FIELD OF THE INVENTION

This invention is directed to flexible packages comprising a co-extruded multi-layer film with a high amount of renewable materials.

### BACKGROUND OF THE INVENTION

Flexible packages made of a co-extruded multi-layer film which comprises a certain amount of renewable materials have already been proposed in order to reduce the use of petroleum-based products such as polyolefins. For example, flexible packages comprising a co-extruded three-layer polymeric film wherein a portion of the polymer in the central layer is replaced by thermoplastic starch have been developed. In view of the rising cost of petroleum, there is still a need of increasing the proportions of renewable raw materials in flexible packages. Therefore, the inventors have developed a new flexible package comprising a multi-layer film. The multi-layer film comprises a print layer, a sealing layer and a core layer, the core layer being sandwiched between the print layer and the sealing layer. Each of the print layer, sealing layer and core layer comprises one or more thermoplastic polymer(s) and thermoplastic starch. The package is described in co-pending European Patent Application No. 13165952.6.

It has been noticed that due to the high thermoplastic starch content of the multi-layer film, the film may have a dull surface. However, the surface of the sealing layer which is the layer of the multi-layer film which is typically in contact with the product(s) which are comprised by the package must be very smooth in order for the flexible package to be manufactured at high speed. Indeed, during the packaging process, a prefabricated bag is typically opened, and a bundle of products, which are often compressed, is then pushed into the open bag. During this latest step, the products are sliding along the bag inner surface. If the inner surface of the multi-layer film is not slick enough, the desired high packaging speed is not attained, resulting in problems such as wrinkled, unclosed, or torn bags.

It is known to use polymer mixtures that contain slip agent that migrate to the inner surface of the film and form a sliding layer there. However, since the migration is a function of time, temperature, and pressure, migrating slip agents may also cause problems due to the greatly fluctuating conditions. Although slip agents are known that do not migrate, they are less effective than migrating slip agents.

When slip agents containing thermoplastic starch as a mixture component are used in polymer mixtures, another problem results. The inventors have discovered that the addition of a slip agent to the sealing layer of the multi-layer film of the newly developed flexible package in a quantity customary for the sealing layer does not result in the desired improvement in sliding quality at the inner surface of the flexible package. Furthermore, if a too high amount of slip agent is added to the sealing layer, this may have a negative impact on the weld strength of the package which in such a case may be reduced. Furthermore, increasing too much the amount of slip agent comprised in the sealing layer may lead to an increased risk of deposits forming at the nozzle gap on the extrusion nozzle during extrusion of the film and also have the disadvantage that the weld strength of the package may decrease.

Hence, a yet unsolved problem lies in the provision of a flexible package made of a multi-layer film which comprises a high amount of renewable raw materials and which comprises an inner surface which exhibits good sliding and sealability properties.

The inventors have surprinsingly found that such a problem my be solved by the provision of a flexible package comprising the newly developed co-extruded multi-layer film, wherein slip agent is added not only to the sealing layer but also to the core layer.

### SUMMARY OF THE INVENTION

The invention relates to a flexible package comprising a co-extruded multi-layer film, the multi-layer film comprising a print layer, a sealing layer and a core layer, the core layer being sandwiched between the print layer and the sealing layer. The package comprises one or more product(s). Each of the print layer, sealing layer and core layer comprises one or more thermoplastic polymer(s) and thermoplastic starch and each of the sealing layer and core layer comprises a slip agent.

### DETAILED DESCRIPTION OF THE INVENTION

"Flexible package" is used herein to refer to a package which is made of flexible or easily yielding materials that, when filled and closed can be readily changed in shape. Examples of flexible packages include, but are not limited to, bags, pouches, sachets, wraps or envelopes made of materials such as paper, plastic film, foil, and combinations thereof.

"Absorbent article" is used herein to refer to devices that absorb and contain body exudates, and, more specifically, refers to devices that are placed against or in proximity to the body of the wearer to absorb and contain the various exudates discharged from the body. Absorbent articles include diapers, training pants, adult incontinence undergarments, feminine hygiene products and the like such as catamenial pads, interlabial pads, panty liners, pessaries, sanitary napkins, tampons and tampon applicators. As used herein, the term "body fluids" or "body exudates" includes, but is not limited to, urine, blood, vaginal discharges, breast milk, sweat and fecal matter. The absorbent article may be a diaper or training pant.

The terms "comprise" or "comprising" also includes the terms "consist of" or "consisting of".

"Print layer" is used herein to refer to the layer of the co-extruded multi-layer film of the package which is oriented toward the outside of the package. The print layer may be printed with graphics such as artworks, trademarks, logos and/or regulatory information.

"Sealing layer" is used herein to refer to the layer of the co-extruded multi-layer film of the package which is oriented toward the inside of the package. The sealing layer is typically in contact with the product(s) which are comprised by the package.

"Inner surface" is used herein to refer to the surface of the co-extruded multi-layer film of the package which is oriented toward the inside of the package and is typically in contact with the product(s) which are comprised by the package and "outer surface" is used herein to refer to the surface of the co-extruded multi-layer film of the package which is oriented toward the outside of the package and which may be may be printed with graphics such as artworks, trademarks, logos and/or regulatory information.

"Renewable resource" is used herein to refer to a resource that is produced by a natural process at a rate comparable to its rate of consumption (e.g., within a 100 year time frame). The resource can be replenished naturally or via engineered agricultural techniques. Non-limiting examples of renewable resources include plants (e.g., sugar cane, beets, corn, potatoes, citrus fruit, woody plants, lignocellulosics, hemicellulosics, cellulosic waste), animals, fish, bacteria, fungi, and forestry products. These resources can be naturally occurring, hybrids, or genetically engineered organisms. Natural resources such as crude oil, coal, natural gas, and peat, which take longer than 100 years to form, are not considered renewable resources.

"Bio-based" is used herein to refer to a component of the multi-layer film that can be produced or is derived from a renewable resource.

"Bio-based content" refers to the amount of carbon from a renewable resource in a material as a percent of the mass of the total organic carbon in the material, as determined by ASTM D6866-10, method B. Note that any carbon from inorganic sources such as calcium carbonate is not included in determining the bio-based content of the material.

To determine the level of bio-based materials present in an unknown composition (e.g., in a product made by a third party), ASTM D6866-10, test method B can be used to measure the bio-based content by measuring the amount of carbon-14 in the product. Materials that come from biomass (i.e. renewable sources) have a well-characterized amount of carbon-14 present, whereas those from fossil sources do not contain carbon-14. Thus, the carbon-14 present in the product is correlated to its bio-based content. Assessment of the materials described herein is done in accordance with ASTM D6866-10, test method B. The mean values of the testing encompass an absolute range of 6% (plus and minus 3% on either side of the bio-based content value) to account for variations in end-component radiocarbon signatures.

In order to apply the methodology of ASTM D6866-10 to determine the bio-based content of a flexible package, a representative sample must be obtained for testing. In one embodiment, a representative portion of the flexible package can be obtained by cutting a 1 cm² portion of the flexible package and grinding it into particulates less than about 20 mesh using known grinding methods (e.g., Wiley® mill), and a representative sample of suitable mass taken from the randomly mixed particles.

"Biodegradable" refers generally to a material that can degrade from the action of naturally occurring microorganisms, such as bacteria, fungi, yeasts, and algae; environmental heat, moisture, or other environmental factors. If desired, the extent of biodegradability may be determined according to ASTM Test Method 5338.92.

"Compatibilizer" means an additive that, when added to a blend of immiscible polymers, modifies their interfaces and stabilizes the blend.

The flexible package according to the present invention comprises a co-extruded multi-layer film. The multi-layer film comprises a print layer, a sealing layer and a core layer, the core layer being sandwiched between the print layer and the sealing layer. The package comprises one or more product(s). Each of the print layer, sealing layer and core layer comprises one or more thermoplastic polymer(s) and thermoplastic starch.

### Slip agent

Each of the sealing layer and core layer comprises a slip agent. The inventors have surprisingly found that a multi-layer film comprising thermoplastic starch in each of its layers may have an inner surface which exhibits good sliding properties, i.e. low coefficient of friction if the slip agent is added not only to the sealing layer but also to the core layer. Whilst not wishing to be bound by theory, it is believed that this is due to the fact that starch has a high affinity for the slip agent, e.g. a fatty acid amide and therefore the thermoplastic starch comprised by the core layer may hinder migration of the slip agent to the surface of the sealing layer. Adding a certain amount of slip agent to the core layer may help to saturate the core layer with slip agent and therefore may reduce the migration of slip agent from the sealing layer to the core layer. Therefore, the distribution of the slip agent over the sealing layer and the core layer allows a reduction of the amount of slip agent in the sealing layer since the slip agent present in the sealing layer is more effective for reducing the coefficient of friction of the inner surface of the multi-layer film. Due to the lower slip agent fraction in the sealing layer, formation of deposits at the nozzle gap during co-extrusion may be prevented, and the sealability of the sealing layer may be improved. As a result, a flexible package comprising a co-extruded multi-layer film according to the present invention is typically characterized by a high weld strength. The lower amount of slip agent in the sealing layer also ensures that no slip agent or only a limited amount of slip agent is transferred from the sealing layer to the print layer when the film is being rolled up.

Furthermore, the outer surface of the multi-layer film according to the present invention may be duller than the inner surface of the multi-layer of the film. This may facilitate the adhesion of printing ink to the print layer as well as adhesive in embodiments wherein film strips, for example are laminated on the outer surface of the multi-layer film.

The sealing layer of the multi-layer film of the package according to the present invention may have a static coefficient of friction of 0.40 or less, or 0.25 or less, or 0.20 or less as measured according to the ISO 8295 test method.

The total amount of slip agent comprised in the sealing layer may be the same as the total amount of slip agent comprised in the core layer. Alternatively, the total amount of slip agent comprised in the sealing layer may be higher than the total amount of slip agent comprised in the core layer. The total amount of slip agent comprised in the core layer may be at least 20%, preferably 30% to 70% of the total amount of slip agent comprised in the sealing layer.

The print layer may be free of slip agent.

The total amount of slip agent comprised in the sealing layer may be less than 90,000 ppm*µm, or from 30,000 ppm*µm to 90,000 ppm*µm For example, the total amount of slip agent comprised in a sealing layer having a thickness of 15 µm and comprising 2000 ppm of slip agent is 30,000 ppm*µm The total amount of slip agent comprised in the sealing layer may be from 1000 ppm to 6000 ppm.

The total amount of slip agent comprised in the core layer may be less than 90,000 ppm*µm, or from 15,000 ppm*µm to 90,000 ppm*µm. For example, the total amount of slip agent comprised in a core layer having a thickness of 30 µm and comprising 500 ppm of slip agent is of 15,000 ppm*µm The total amount of slip agent comprised in the core layer may be higher than 500 ppm but lower than the total amount of slip agent comprised in the sealing layer.

### Type of slip agents

The slip agent may be a fatty acid amide. The fatty acid amide may be selected from erucic acid amide, oleic acid amide, stearic acid amide and combinations thereof. The fatty acid amide may be erucic acid amide.

### Thermoplastic starch (TPS)

Since each of the print layer, sealing layer and core layer of the multi-layer film comprises thermoplastic starch, the three layers are therefore characterized by similar or essentially identical flow properties and elongation properties so that no instabilities can occur during the co-extrusion of the individual layers. Melt fracture, uneven surfaces or fluctuations in thickness of the film may typically be prevented. Due to this more symmetrical layer structure, the multi-layer film comprised by the package is characterized in particular by a high strength and good resistance to delamination.

The total amount of thermoplastic starch comprised in the core layer may be higher than the total amount of thermoplastic starch comprised in the print layer and/or the total amount of thermoplastic starch comprised in the sealing layer. The thermoplastic starch content of the core layer may be up to twice as high as the thermoplastic starch content of the print layer and/or the thermoplastic starch content of the sealing layer. This may be particularly advantageous from a process point of view. Indeed, during the extrusion process, contact between a layer having a high content of thermoplastic starch such as the core layer and the metal surfaces of the nozzle of the blow head of the extruder could lead to the development of deposits on the nozzle, which would disturb the production of the multilayer film. Therefore, since the core layer is separated from the metal surfaces of the nozzle by the print layer and sealing layer which both have a lower content of thermoplastic starch, the formation of deposits, which may be caused by the high thermoplastic starch content of the core layer may be reduced or even prevented. Furthermore, the print layer can be easily printed and reliable sealability of the film may be ensured by the sealing layer.

The core layer may comprise from 5% to 50%, or from 5% to 30%, or from 5% to 20%, or from 15% to 20% by weight of a total amount of thermoplastic starch.

The total amount of thermoplastic starch comprised in the print layer may be the same as or higher than the total amount of thermoplastic starch comprised in the sealing layer. Having a lower thermoplastic starch content for the sealing layer than for the print layer is particularly advantageous since a reliable sealability of the film may therefore be ensured by the sealing layer. The print layer and/or the sealing layer may comprise from 2% to 25%, or from 2% to 20%, or from 3% to 12% by weight of a total amount of thermoplastic starch.

The print layer may comprise from 8% to 12% by weight of a total amount of thermoplastic starch and the sealing layer may comprise from 2% to 6% of a total amount of thermoplastic starch.

Starch: As used herein, "starch" means a native starch or a starch derivative that has been destructured by thermomechanical treatment with one or more plasticizers that can optionally be removed (e.g. water). As used herein, "thermoplastic starch" or "TPS" means a native starch or a starch derivative that has been rendered destructured and thermoplastic by treatment with one or more plasticizers, with at least one plasticizer still remaining. Thermoplastic starch compositions are well known and disclosed in several patents, for example: U.S. Patent Nos. 5,280,055; 5,314,934; 5,362,777; 5,844,023; 6,214,907; 6,242,102; 6,096,809; 6,218,321; 6,235,815; 6,235,816; and 6,231,970.

Since natural starch generally has a granular structure, it needs to be destructurized before it can be melt processed like a thermoplastic material. For gelatinization, e.g., the process of destructuring the starch, the starch can be destructurized in the presence of a solvent which acts as a plasticizer. The solvent and starch mixture is heated, typically under pressurized conditions and shear to accelerate the gelatinization process. Chemical or enzymatic agents may also be used to destructurize, oxidize, or derivatize the starch. Commonly, starch is destructured by dissolving the starch in water. Fully destructured starch results when the particle size of any remaining undestructured starch does not impact the extrusion process, e.g., a fiber spinning process or a film-forming process. Any remaining undestructured starch particle sizes are less than 30µm (by number average), preferably less 15µm, more preferably less than 5µm, or less than 2µm The residual particle size can be determined by pressing the final formulation into a thin film (50µm or less) and placing the film into a light microscope under cross polarized light. Under cross polarized light, the signature maltese cross, indicative of undestructured starch, can be observed. If the average size of these particle is above the target range, the destructured starch has not been prepared properly. An alternative process for measuring the amount and size of undestructured starch is by means of a melt filtration test in which a composition containing the starch is passed through a series of screens that can capture residual undestructured starch.

Suitable naturally occurring starches can include, but are not limited to, corn starch, potato starch, sweet potato starch, wheat starch, sago palm starch, tapioca starch, rice starch, soybean starch, arrow root starch, bracken starch, lotus starch, cassaya starch, waxy maize starch, high amylose corn starch, and commercial amylose powder. Blends of starch may also be used. Though all starches are useful herein, the present invention is most commonly practiced with natural starches derived from agricultural sources, which offer the advantages of being abundant in supply, easily replenishable and inexpensive in price. Naturally occurring starches, particularly corn starch, wheat starch, and waxy maize starch, are the preferred starch polymers of choice due to their economy and availability.

Modified starch may also be used. Modified starch is defined as non-substituted or substituted starch that has had its native molecular weight characteristics changed (i.e. the molecular weight is changed but no other changes are necessarily made to the starch). If modified starch is desired, chemical modifications of starch typically include acid or alkali hydrolysis and oxidative chain scission to reduce molecular weight and molecular weight distribution. Natural, unmodified starch generally has a very high average molecular weight and a broad molecular weight distribution (e.g. natural corn starch has an average molecular weight of up to 60,000,000 grams/mole (g/mol)). The average molecular weight of starch can be reduced to the desirable range for the present invention by acidic, oxidative cleavage, enzymatic reduction, hydrolysis (acid or alkaline catalyzed), physical/mechanical degradation (e.g., via the thermomechanical energy input of the processing equipment), or combinations thereof. The thermomechanical method and the oxidative method offer an additional advantage when carried out in situ. The exact chemical nature of the starch and molecular weight reduction method is not critical as long as the average molecular weight is in an acceptable range.

Ranges of number average molecular weight for starch or starch blends added to the melt can be from 3,000 g/mol to 20,000,000 g/mol, or from 10,000 g/mol to 10,000,000 g/mol, or from 15,000 to 5,000,000 g/mol, or from 20,000 g/mol to 3,000,000 g/mol. In other embodiments, the average molecular weight is otherwise within the above ranges but about 1,000,000 or less, or about 700,000 or less.

Substituted starch can be used. If substituted starch is desired, chemical modifications of starch typically include etherification and esterification. Substituted starches may be desired for better compatibility or miscibility with the thermoplastic polymer and plasticizer. Alternatively, modified and substituted starches can be used to aid in the destructuring process by increasing the gelatinization process. However, this must be balanced with the reduction in the rate of degradability. The degree of substitution of the chemically substituted starch is generally from 0.01 to 3.0, with some having a low degree of substitution, such as 0.01 to 0.06.

Plasticizer: A plasticizer can be used in the present invention to destructurize the starch and enable the starch to flow, i.e. create a thermoplastic starch. The same plasticizer may be used to increase melt processability or two separate plasticizers may be used. The plasticizers may also improve the flexibility of the final films, which is believed to be due to the lowering of the glass transition temperature of the composition of the film layers by the plasticizer. The plasticizers should preferably be substantially compatible with the polymeric components of the disclosed compositions of the film layers so that the plasticizers may effectively modify the properties of the film layers. As used herein, the term "substantially compatible" means when heated to a temperature above the softening and/or the melting temperature of the composition, the plasticizer is capable of forming a substantially homogeneous mixture with starch.

An additional plasticizer or diluent for the thermoplastic polymer may be present to lower the polymer's melting temperature and improve overall compatibility with the thermoplastic starch blend. Furthermore, thermoplastic polymers with higher melting temperatures may be used if plasticizers or diluents are present which suppress the melting temperature of the polymer. The plasticizer will preferrably have a molecular weight of less than 300,000g/mol, more preferably less than 200,000g/mol and most preferably less 100,000 g/mol and may preferably be a block or random copolymer or terpolymer where one or more of the monomer(s) is compatible with another plasticizer, starch, polymer, or combinations thereof. Non-limiting examples of polymeric starch plasticizers include ethylene vinyl alcohol and polyvinyl alcohol.

Nonlimiting examples of useful hydroxyl plasticizers include sugars such as glucose, sucrose, fructose, raffinose, maltodextrose, galactose, xylose, maltose, lactose, mannose erythrose, glycerol, and pentaerythritol; sugar alcohols such as erythritol, xylitol, malitol, mannitol and sorbitol; polyols such as ethylene glycol, propylene glycol, dipropylene glycol, butylene glycol, hexane triol, and the like, and polymers thereof; and mixtures thereof. Also useful herein as hydroxyl plasticizers are poloxomers and poloxamines. Also suitable for use herein are hydrogen bond forming organic compounds which do not have hydroxyl group, including urea and urea derivatives; anhydrides of sugar alcohols such as sorbitan and isosorbide; animal proteins such as gelatin; vegetable proteins such as sunflower protein, soybean proteins, cotton seed proteins; and mixtures thereof. Other suitable plasticizers are phthalate esters, dimethyl and diethylsuccinate and related esters, glycerol triacetate, glycerol mono and diacetates, glycerol mono, di, and tripropionates, and butanoates, which are biodegradable. Copolymers such as ethylene acrylic acid, ethylene maleic acid, butadiene acrylic acid, butadiene maleic acid, propylene acrylic acid, propylene maleic acid, and other copolymers ofunsaturated hydrocarbon based acids can also be used. All of the plasticizers may be use alone or in mixtures thereof.

Preferred plasticizers include glycerin, mannitol, and sorbitol, with sorbitol being the most preferred. The amount of plasticizer is dependent upon the molecular weight, amount of starch, and the affinity of the plasticizer for the starch. Generally, the amount of plasticizer increases with increasing molecular weight of starch.

The thermoplastic starch may comprise from 60% to 90% by weight of a total amount of starch.

### Thermoplastic polymer(s)

Each of the print layer, sealing layer and core layer of the multi-layer film comprises one or more thermoplastic polymer(s).

Thermoplastic polymer(s), as used herein, are polymers that melt and then, upon cooling, crystallize or harden, but can be re-melted upon further heating. Suitable thermoplastic polymer(s) used herein have a melting temperature from 60°C to 300°C, from 80°C to 250°C, or from 100°C to 215°C.

Thermoplastic polymer(s) can be bio-based or derived from fossil-based materials. Thermoplastic polymer(s) may be bio-based, for example such as bio-produced ethylene and propylene monomers used in the production of polypropylene and polyethylene. These material properties are essentially identical to fossil-based product equivalents, except for the presence of carbon-14 in the bio-based thermoplastic polymer(s).

Bio-based and fossil-based thermoplastic polymers can be combined together in the present invention in any ratio, depending on cost and availability. Recycled thermoplastic polymers can also be used, alone or in combination with bio-based and/or fossil-based thermoplastic polymers.

For example, the thermoplastic polymers can comprise greater than 10% bio-based material, or greater than 50%, or from 30% to 100%, or from 1% to 100% bio-based material, based upon the total weight of thermoplastic polymer(s) present.

Suitable thermoplastic polymers can have weight average molecular weights of 1000 kDa or less, 5 kDa to 800 kDa, 10 kDa to 700 kDa, or 20 kDa to 400 kDa. The weight average molecular weight is determined by the specific ASTM method for each polymer, but is generally measured using gel permeation chromatography (GPC).

The thermoplastic polymer(s) may be selected from polyolefins, polyesters, polyamides, copolymers thereof, and combinations thereof.

### Polyolefin(s)

Each of the print layer, sealing layer and core layer may comprise polyolefin(s) selected from polypropylene, polyethylene, copolymers thereof, and combinations thereof.

### Polypropylene:

The print layer and/or the sealing layer and/or the core layer may comprise polypropylene and/or copolymer(s) thereof.

Polypropylene may be selected from atactic polypropylene, isotactic polypropylene, syndiotactic polypropylene, and combinations thereof.

Polypropylene copolymer(s) may be selected from polypropylene random copolymer (PP-RC) and/or polypropylene block copolymer (PP-BC). The polypropylene block copolymer (PP-BC) may be formed from propylene and at least one α-olefin. The α-olefin may be selected from 1-butene, 1-hexene and 1-octene and combinations thereof.

The polypropylene and/or copolymer(s) thereof may have a melt flow index (MFI) of more than 1, as measured according to DIN 53735, test conditions 230°C, 2.16 kg.

The core layer may comprise one or more polypropylene(s) and/or copolymer(s) thereof. Due to the use of polypropylene and/or copolymer(s) thereof in the core layer, the core layer and thus also the film are characterized by excellent tensile strength.

The core layer may comprise a polypropylene block copolymer (PP-BC) and/or a polypropylene random copolymer (PP-RC). The adhesion between the individual print layer, sealing layer and core layers and the flow behavior of the core layer are improved by the PP-RC. The tensile strength of the film is increased by the PP-BC.

The core layer may comprise from 15% to 60%, or from 20% to 50% by weight of a total amount of polypropylene and/or copolymer(s) thereof.

The core layer may comprise from 5% to 30%, or from 10% to 20% by weight of a total amount of polypropylene random copolymer (PP-RC) and/or from 15% to 40%, or from and 20% to 30% by weight of a total amount of polypropylene block copolymer (PP-BC).

The print layer and/or the sealing layer may comprise one or more polypropylene and/or copolymer(s) thereof.

The print layer and/or the sealing layer may comprise a polypropylene random copolymer (PP-RC).

The PP-RC may advantageously have an ethylene comonomer content of at least 4%, based on the weight of the PP-RC.

It is particularly advantageous to use a polypropylene and/or copolymer(s) thereof, especially a polypropylene random copolymer (PP-RC) in the print layer and/or the sealing layer of the film. Indeed, the adhesion between the print layer and the core layer and/or the sealing layer and the core layer is improved and therefore the risk of delamination of the different layers of the film is also reduced.

The print layer and/or the sealing layer(s) may comprise from 5% to 50% or from 10% to 35% by weight of a total amount of polypropylene and/or copolymer(s) thereof.

The print layer and/or the sealing layer comprise(s) from 10% to 35% by weight of a total amount of polypropylene random copolymer (PP-RC)

### Polyethylene:

The print layer and/or the sealing layer and/or the core layer may comprise polyethylene and/or copolymer(s) thereof.

Polyethylene and/or copolymer(s) thereof may be selected from low density polyethylene (LDPE), linear low density polyethylene (LLDPE), metallocene-catalyzed linear low density polyethylene (mLLDPE) and combinations thereof.

mLLDPE may be used with 1-hexene as the comonomer also known as mLLDPE-C6. It is also possible for α-olefins such as 1-butene or 1-octene to be added as a comonomer to the mLLDPE.

The core layer may comprise from 5% to 40%, or from 15% to 25% by weight of a total amount of polyethylene and/or copolymer(s) thereof.

The core layer may comprise metallocene-catalyzed linear low density polyethylene (mLLDPE). The core layer may comprise from 10% to 40% by weight of a total amount of metallocene-catalyzed linear low density polyethylene (mLLDPE).

It is particularly advantageous to use polyethylene, especially mLLDPE in the core layer. Indeed, by having polyethylene in the core layer, the tensile strength of the core layer is improved and the tear propagation resistance of the core layer and thus of the film is increased.

The melt flow index MFI of the polyethylene may be more than 1.5 (measured according to DIN 53735, test conditions 190°C, 2.16 kg).

The print layer and/or the sealing layer may comprise from 40% to 90%, or from 50% to 85% by weight of a total amount of polyethylene and/or copolymer(s) thereof.

The polyethylene and/or copolymer(s) thereof comprised in the print layer and/or the sealing layer may be selected from Low Density Polyethylene (LDPE), Linear Low Density Polyethylene (LLDPE), metallocene-catalyzed Linear Low Density Polyethylene (mLLDPE) and combinations thereof.

Having polyethylene and/or copolymer(s) thereof in the sealing layer ensures the sealing capacity of the film.

### Compatibilizer(s)

The print layer and/or the sealing layer and/or the core layer may comprise one or more compatibilizer(s). Compatibilizer(s) may be used to improve the compatibility and dispersion characteristics of TPS in polyolefins. Several compatibilizers with both polar and non-polar groups may be comprised by the print layer and/or the sealing layer and/or the core layer of the multi-layer film of the package.

The compatibilizer(s) may be selected from a non-polar backbone and a grafted polar functional monomer, a block copolymer of a both a non-polar block and a polar block, a non-polymeric polar material, a non-polar material and combinations thereof.

The compatibilizer(s) may be selected from polyethylene-co-vinyl acetate (EVA), polyethylene-co-butyl acrylate (EBA), polyethylene-co-vinyl alcohol (EVOH), polyethylene-co-acrylic acid (EAA), a graft copolymer of a polyolefin (e.g., polyethylene or polypropylene) and maleic anhydride, and combinations thereof. The compatibilizer(s) may be selected from a graft copolymer of polypropylene and maleic anhydride (PP-g-MA), and/or a graft copolymer of polyethylene and maleic anhydride (PE-g-MA).

EVA, EBA, EVOH, and EAA have a non-polar polyethylene subunit in their backbone. The vinyl acetate and butyl acrylate subunit contains an ester group, which associate with the hydroxyl groups of the amylopectin and amylase of the starch molecules. Instead of the ester group from the vinyl acetate subunit, EVOH has a vinyl alcohol group which has hydroxyl group as in the starch molecules. Both the ester group in EVA and EBA and the hydroxyl group in EVOH do not chemically react with the hydroxyl groups starch molecules. They only associate with starch through hydrogen bonding or polar-polar molecular interactions. Using these two physical compatibilizers, TPS and EVA or EBA or EVOH blends showed improved compatibility versus the uncompatibilized PE/TPS blends.

In the structure of the graft copolymer of polypropylene (or polyethylene) and maleic anhydride (PP-g-MA or PE-g-MA), the cyclic anhydride at one end is chemically bonded directly into the polypropylene (or polyethylene) chain. The polar anhydride group of the molecule could associate with the hydroxyl groups in the starch via both hydrogen bonding and polar-polar molecular interactions and a chemical reaction to form an ester linkage during the melt extrusion process. The hydroxyls of the starch can undergo esterification reaction with the anhydride to achieve a ring-opening reaction to chemically link the TPS to the maleic anhydride that is grafted to polypropylene (or polyethylene). This reaction is accomplished under the high temperatures and pressures of the extrusion process.

PP-g-MA or PE-g-MA such as the ones sold under the following tradenames may be used: DuPont Fusabond^{®} , Mitsui Admer^{®}, Arkema Orevac^{®}, Equistar Plexar^{®}, DuPont Bynel^{®}, Manas Optim^{®} or Exxon Exxelor^{®}.

The print layer and/or the sealing layer and/or the core layer may each comprise from 1% to 15% by weight of compatibilizer(s).

The total amount of TPS and compatibilizer, respectively, present in the print layer and/or the sealing layer and/or the core layer can be expressed as a ratio of between about 10:1 to 2:1 or 7:1 to 3:1.

### Additional additives

The core layer may comprise at least one additional additive. The additional additive may be selected from a pigment, a dye, a dye mixture and combinations thereof. The print layer and/or the sealing layer may be advantageously designed to be free of additives, in particular free of pigments and/or coloring agents. Hence, during the extrusion process, the core layer which comprises the additive(s) is separated from the metal surfaces of the nozzle of the blow head for producing the multilayer film. This reduces the risk of the formation of deposit on the metal surfaces of the nozzle.

### Bio-based content

The multi-layer film of the package may comprise a bio-based content value from 5% to 90%, or from 10% to 90%, or from 10% to 35% measured according to ASTM D6866-10, method B. The components of the film which may be bio-based include thermoplastic starch (TPS) including plasticizers (such as glycerin, mannitol, and sorbitol). Polyethylene, polypropylene and/or copolymer(s) thereof comprised in the different layers of the film may also be bio-based.

### Layer Thickness

The multi-layer film of the package may have a thickness of from 10 µm to 200 µm, or from 30 µm to 100 µm The multi-layer film of the package may have a thickness of approximately 50 µm. The thickness of the film may be measured according to the ISO 4593 test method.

The print layer and the sealing layer may have the same thickness. If higher mechanical strengths are required, the core layer may also be thicker than each of the print layer and sealing layer. The print layer/core layer/sealing layer thickness ratio may be from 2:1:2 to 1:4:1.

The core layer may have a thickness of from 10 µm to 50 µm, or from 20 µm to 40 µm.

Each of the print layer and the sealing layer may have a thickness of from 5 µm to 50 µm, or from 10 µm to 25 µm.

### Product(s)

The flexible package comprise one or more product(s).

The product(s) may be selected from absorbent article(s), bibs, personal care products including hand soaps, shampoos, lotions, oral care implements, clothing and combinations thereof.

The product(s) may also be selected from products for treating hair (human, dog, and/or cat) including: bleaching, coloring, dyeing, conditioning, growing, removing, retarding growth, shampooing, styling; deodorants and antiperspirants; personal cleansing; color cosmetics; products for treating skin (human, dog, and/or cat) including: creams, lotions, and other topically applied products; orally administered products for enhancing the appearance of hair, skin, and/or nails (human, dog, and/or cat); shaving products and combinations thereof.

The product(s) may also be selected from products for treating fabrics, products for treating and/or cleaning hard surfaces, air care products, car care products, dishwashing products, fabric conditioning (including softening) products, laundry detergent and combinations thereof.

The product(s) may also be selected from wet or dry bath tissue, facial tissue, disposable handkerchiefs, disposable towels, wipes and combinations thereof.

The product(s) may also be selected from compositions for use with any soft and/or hard tissue of the oral cavity or conditions associated therewith (e.g., anti-caries compositions, anti-microbial compositions, anti-plaque chewing gum, compositions, breath compositions, confectionaries, dentifrices, denture compositions, lozenges, rinses, and tooth whitening compositions), cleaning devices, floss and flossing devices, toothbrushes; cough and cold remedies and treatments for other respiratory conditions, pain relievers whether topical, oral, or otherwise, gastrointestinal remedies including any composition suitable for the alleviation of gastrointestinal conditions such as heartburn, upset stomach, diarrhea, and irritable bowel syndrome, and nutrient supplementation such as calcium or fiber supplementation; pet health and nutrition including pet foods, treats; topical products such as grooming aids, training aids, devices, toys; waters including purified, flavored, or other treated waters and combinations thereof.

The flexible package may comprise a single product. The single product may be an absorbent article. The absorbent article may be a feminine hygiene product such as a sanitary napkin or a panty liner. The absorbent article comprises a body facing side, a garment facing side, two longitudinal sides and two transverse sides. The multi-layer film of the flexible package may be in the form of a wrapper which overlays the garment facing side of the absorbent article. The wrapper may extend beyond the perimeter of the absorbent article so that when the absorbent article and the wrapper are folded as a unit, the longitudinal side flaps of the wrapper, which extend beyond the longitudinal sides of the article, may be frangibly sealed thereby providing the absorbent article with an individual package.

It is also common to provide the absorbent article with an adhesive element on the garment facing side which, in use, serves to affix the absorbent article to the wearer's undergarment thereby maintaining the absorbent article in place against the wearer's body. The adhesive element may take the form of a coating of adhesive which is in strips or other suitable pattern. For example, the garment facing side of the absorbent article can be coated uniformly with a layer of pressure sensitive hot melt adhesive. The wrapper may overlays the garment facing side of the absorbent article with the longitudinal flap portions extending beyond the longitudinal perimeter segments of the absorbent article. The wrapper is typically not folded onto or otherwise brought into contact with the body facing side of the absorbent article. The wrapper is typically releasably affixed to the absorbent article, e.g. a sanitary napkin, by the aforementioned adhesive element. When an adhesive element is used in this manner, it is not necessary to provide the absorbent article with a separate release sheet in order to protect the adhesive element before use, as this function is provided by the wrapper.

To individually package the absorbent article, the absorbent article and the affixed wrapper may be typically folded as a unit. That is, they are folded together with the wrapper remaining in place with respect to the absorbent article. Typically, the absorbent article is folded lengthwise into thirds about two fold axes. The longitudinal side flaps or flap portions of the wrapper are frangibly sealed using any of the well known sealing techniques. For example, the longitudinal flap portions may be heat sealed, glued, ultrasonically bonded, or crimped.

In use, the individually packaged absorbent article is provided to a user. The user may then break the frangible seals, unfold the wrapper/absorbent article unit and separate the wrapper from the absorbent article, for example a sanitary napkin, exposing the adhesive element. The absorbent article may then be used as such devices normally are, typically being adhered by means of the adhesive element to the crotch portion of an undergarment, which is subsequently worn. Individually packaged absorbent articles are disclosed, for example in US patent 4,556,146, PCT patent application WO2008/075290, US patent applications US 2009/0240227A1 and US 2011/0202028A1.

### Examples

In the following section all the percentages are percentages by weight (wt%).

Three different multi-layer films are prepared : a multi-layer film according to the present invention (Example 1), wherein a slip agent is added to both the sealing layer and the core layer and two multi-layer films (Comparative examples 1 and 2) wherein the slip agent is added to only the sealing layer.

Each of these films is produced as a co-extruded blown film, wherein each of the layers of the films has a thickness of 20 µm. The two multi-layer fims were prepared on 3 layer blown film line using an extruder with L/D ratio of 30:1, BUR (blow up ratio) = 2-3. The melt temperature was kept below about 190°C to minimise starch disintegration and discoloration. Coefficient of friction measurements were carried out on the inner surface of the films 3 days after manufacturing of the films. The coefficients of friction (COF) were measured in accordance with ISO 8295 test method; the stated measured values refer to the film/film material pairing. The higher the static coefficient of friction, the duller the film.

### • Example 1:

| Components | Print layer | Core layer | Sealing layer |
|---|---|---|---|
| TPS masterbatch¹ | 45 wt% | 53 wt% | 30 wt% |
| Polyethylene (LDPE + LLDPE) | 47 wt% | 22 wt% | 56 wt% |
| White-masterbatch² | 0 wt% | 14 wt% | 0 wt% |
| Slip agent-masterbatch³ (concentration of slip agent in the layer) | | 3 wt% (1500 ppm) | 6 wt% (3000 ppm) |
| Compatibilizer (EVA) | 8 wt% | 8 wt% | 8 wt% |

| | | | |
|---|---|---|---|
| ¹ The thermoplastic starch (TPS) masterbatch comprises 46 wt% of starch, 20 wt% of sorbitol/Glycerol, 17 wt% compatibilizer (EAA), 16 wt% polyolefins and 1 wt% of other additives. ²The white-masterbatch comprises 40% polyethylene and 60% titanium dioxide. ³The slip agent masterbatch comprises 50,000 ppm erucic acid amide in polyethylene. | | | |

### • Comparative Example 1:

| Components | Print layer | Core layer | Sealing layer |
|---|---|---|---|
| TPS masterbatch¹ | 45 wt% | 53 wt% | 30 wt% |
| Polyethylene (LDPE + LLDPE) | 47 wt% | 25 wt% | 56 wt% |
| White-masterbatch² | 0 wt% | 14 wt% | 0 wt% |
| Slip agent-masterbatch³ (concentration of slip agent in the layer) | | | 6 wt% (3000 ppm) |
| Compatibilizer (EVA) | 8 wt% | 8 wt% | 8 wt% |

| | | | |
|---|---|---|---|
| ¹The thermoplastic starch (TPS) masterbatch comprises 46 wt% of starch, 20 wt% of sorbitol/Glycerol, 17 wt% compatibilizer (EAA), 16 wt% polyolefins and 1 wt% of other additives. ²The white-masterbatch comprises 40% polyethylene and 60% titanium dioxide. ³The slip agent masterbatch comprises 50,000 ppm erucic acid amide in polyethylene. | | | |

### • Comparative Example 2:

| Components | Print layer | Core layer | Sealing layer |
|---|---|---|---|
| TPS masterbatch¹ | 45 wt% | 53 wt% | 30 wt% |
| Polyethylene (LDPE + LLDPE) | 47 wt% | 25 wt% | 53 wt% |
| White-masterbatch² | 0 wt% | 14 wt% | 0 wt% |
| Slip agent-masterbatch³ (concentration of slip agent in the layer) | | | 9 wt% (4500 ppm) |
| Compatibilizer (EVA) | 8 wt% | 8 wt% | 8 wt% |

| | | | |
|---|---|---|---|
| ¹The thermoplastic starch (TPS) masterbatch comprises 46 wt% of starch, 20 wt% of sorbitol/Glycerol, 17 wt% compatibilizer (EAA), 16 wt% polyolefins and 1 wt% of other additives. ²The white-masterbatch comprises 40% polyethylene and 60% titanium dioxide. ³The slip agent masterbatch comprises 50,000 ppm erucic acid amide in polyethylene. | | | |

### Results:

| Tested film | Coefficient of friction |
|---|---|
| Example 1 | 0.18 |
| Comparative Example 1 | 0.30 |
| Comparative Example 2 | 0.20 |

### Interpretation of the results:

### • Comparison example 1 vs. comparative example 1

The films of comparative example 1 and example 1 have the same amount of slip agent in the sealing layer. The film of example 1 only differs from the film of comparative example 1 in that the core layer also comprises a certain amount of slip agent. As can be seen from the results, it is possible to reduce the coefficient of friction of the sealing layer by also adding a slip agent to the core layer of the film.

### • Comparison example 1 vs. comparative example 2

The films of example 1 and comparative example 2 have the same total amount of slip agent, namely 4500 ppm. In the film of example 1, the slip agent is distributed between the core layer and the sealing layer whereas in the the film of comparative example 2, the slip agent is only comprised by the sealing layer. As can be seen from the results, the coefficient of friction is more reduced if the slip agent is distributed between the core layer and the sealing layer than when a higher amount of slip agent is comprised in only the sealing layer.

Furthermore, as already explained hereinbefore, having a high amount of slip agent in the sealing layer may also has an adverse effect on the functional properties of the film. Thus, the sealability and the achievable weld strength may be impaired as a result of the high amount of slip agent. In addition, the high amount of slip agent may result in deposits at the nozzle gap during co-extrusion. Last, there is the problem that the high amount of slip agent in the sealing layer may result in the slip agent being transferred to the opposite side of the film when the film is wound up into a roll. This effect is referred to as "pick-off." The printable outer layer may be smooth due to such pick-off, which may result in problems in further processing. For example, flexible packages that are smooth on the outer side may not be able to stack well. In addition, adhesion of printing ink may be impaired if the printable outer layer contains some of the slip agent.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A flexible package comprising a co-extruded multi-layer film, the multi-layer film comprising a print layer, a sealing layer and a core layer, the core layer being sandwiched between the print layer and the sealing layer, wherein the package comprises one or more product(s) and wherein:
a. each of the print layer, sealing layer and core layer comprises one or more thermoplastic polymer(s) and thermoplastic starch and
b. each of the sealing layer and core layer comprises a slip agent.

2. The flexible package according to claim 1, wherein the total amount of slip agent comprised in the sealing layer is the same as or higher than the total amount of slip agent comprised in the core layer.

3. The flexible package according to claim 1 or 2, wherein the total amount of slip agent comprised in the core layer is at least 20%, preferably 30% to 70% of the total amount of slip agent comprised in the sealing layer.

4. The flexible package according to any of the preceding claims, wherein the print layer is free of slip agent.

5. The flexible package according to any of the preceding claims, wherein the total amount of slip agent comprised in the sealing layer is less than 90,000 ppm*µm, preferably from 30,000 ppm*µm to 90,000 ppm*µm.

6. The flexible packaging according to any of the preceding claims, wherein the total amount of slip agent comprised in the core layer is less than 90,000 ppm*µm, preferably from 15,000 ppm*µm to 90,000 ppm*µm.

7. The flexible package according to any of the preceding claims, wherein each of the print layer and the sealing layer has a thickness of from 10 µm to 50 µm, preferably 10 µm to 25 µm and wherein the core layer has a thickness of from 10 µm to 50 µm, preferably 20 µm to 40 µm

8. The flexible package according to any of the preceding claims, wherein the slip agent is a fatty acid amide, preferably a fatty acid amide selected from the group consisting of erucic acid amide, oleic acid amide, stearic acid amide and combinations thereof, preferably erucic acid amide.

9. The flexible package according to any of the preceding claims, wherein:
a. the total amount of thermoplastic starch comprised in the core layer is higher, preferably up to twice as high as the total amount of thermoplastic starch comprised in the print layer and/or sealing layer; and/or
b. The total amount of thermoplastic starch comprised in the print layer is the same or higher than the total amount of thermoplastic starch comprised in the sealing layer.

10. The flexible package according to any of the preceding claims, wherein the
a. the print layer and/or the sealing layer comprise(s) from 2% to 25%, preferably from 2% to 20%, more preferably from 3% to 12% by weight of a total amount of thermoplastic starch; and/or
b. the core layer comprises from 5% to 50% by weight, preferably from 5% to 30%, more preferably from 5% to 20%, even more preferably from 15% to 20% by weight of a total amount of thermoplastic starch.

11. The flexible package according to any of the preceding claims, wherein the thermoplastic polymer(s) is/are polyolefin(s) and wherein the polyolefin(s) are selected from polypropylene, polyethylene, copolymers thereof, and combinations thereof.

12. The flexible package according to any of the preceding claims, wherein the film has a thickness of from 10 µm to 200 µm, or 30 µm to 100 µm

13. The flexible package according to any of the preceding claims, wherein the product(s) is/are absorbent article(s).

14. The flexible package according to any of the preceding claims, wherein the package comprises a single product which is an absorbent article comprising a body facing side, a garment facing side, two longitudinal sides and two transverse sides, wherein the multi-layer film is in the form of a wrapper which overlays the garment facing side of the absorbent article.

15. The flexible package according to any of the preceding claims, wherein the multi-layer film comprises from 5% to 90% of bio-based content measured according to ASTM D6866-10, method B.
